# EUROPEAN PATENT APPLICATION

(11) **EP 1 506 791 A1**
(43) Date of publication of application: **16.02.2005**
(21) Application number: 03728112.8
(22) Date of filing: 20.05.2003
(51) Int. Cl.: A61M 5/32, A61M 5/158

(54) **INJECTION NEEDLE**

(30) Priority: 20.05.2002 JP 2002145353
(71) Applicant: KABUSHIKI KAISHA TOP, Tokyo 120-0035 (JP)
(72) Inventor: SUZUKI, Takashi, c/o KABUSHIKI KAISHA TOP, Tokyo 120-0035 (JP)
(74) Representative: Fenlon, Christine Lesley
(86) International application number: PCT/JP2003/006251
(87) International publication number: WO 2003/097134

(57) **Abstract**

An injection needle by which a direction of a blade face at a needle tip can easily be confirmed when injecting is provided maintaining strength of a needle base. An injection needle 1 comprises a needle tube 2 having a blade face 2a cut along an oblique planar face at a needle tip and a needle base 3 formed in a rear portion of the needle tube. The needle base 3 comprise a flat plate portion 5 disposed in front of the needle base 3, a disc-shaped collar portion 6 attached to a rear end of the flat plate portion 5 and orthogonal to an axial direction of the needle tube 2, and an attaching portion 7 extending rearward from the rear end of the collar portion 6. The flat plate portion 5 further includes an identifying projection 8. The flat plate portion 5, formed into a thin plate, extends vertically along a direction to which the blade face 2a opens. Because twisting the injection needle 1 is made easier by holding the flat plate portion, a user can easily add power when attaching the injection needle 1 to an injector. When injecting, a user can easily identify the direction of the blade face 2a by means of the identifying projection 8 of the needle base 3.

## Description

### Technical Field

The present invention relates to an injection needle used for injection or for collection of blood or the like in a clinical site or another situation.

### Background Art

An injection needle which has heretofore often been used has a needle tube (cannula) and a substantially cylindrical needle base, and the needle tube is cut in an obliquely planar state to form a blade face. When a user injects a drug solution by use of such an injection needle, the injection needle is attached to an injector and then inserted into an affected part. At this time, the blade face is set to an upward state to smoothly inject the drug solution. For this reason, the blade face has been upward directed at the time of the injection.

However, in the conventional injection needle, both the needle tube and the needle base are substantially cylindrical, and therefore it is necessary to visually confirm the blade face direction of the needle tube before the injection. However, the blade face of the needle tube is small, and hence it is difficult to confirm the direction.

On the other hand, Japanese Patent Laid-open Publication No. 60-156473 has disclosed an injection needle having a needle tube with a blade face and a needle base disposed at a rear portion of the needle tube. The needle base of this kind of injection needle has a flat portion which shows a direction of the blade face, and this flat portion permits a user to easily identify the direction of the blade face.

However, in the injection needle disclosed in the above patent publication, the flat plane is formed in a substantially cylindrical portion at which the needle base is attached to an injector. Such a substantially cylindrical portion needs strength to some extent, because a tip of the injector is inserted thereinto. However, the flat plane is formed by cutting off a portion of the cylindrical portion of the needle base. Accordingly, if a large area is taken for the flat plane, there is a disadvantage that a portion attached to the injector becomes thinner, with the result that the strength lowers. On the contrary, if the strength is maintained, there is another disadvantage that the area of the flat plane becomes small and therefore the blade face cannot be easily identified.

The present invention has been developed to improve an injection needle and to thereby resolve the above-mentioned disadvantages, and the object of the present invention is to provide an injection needle in which a user can easily confirm a direction of a blade face of a needle tube at the time of injection, without lowering strength of a needle base.

### Disclosure of the Invention

In order to attain the above object, an injection needle according to a first embodiment of the present invention comprises a needle tube having an oblique blade face formed at a tip thereof, and a needle base fixed to a base portion of the needle tube, wherein the needle base includes an attaching portion to be attached to an injector or the like, and a flat plate portion of substantially flat plate shape disposed in front of the attaching portion, the flat plate portion is extended in accordance with upper and lower directions of the blade face, and an identifying shape portion which enables a user to tactually identify the direction of the blade face is formed on a side edge of the flat plate portion.

According to the injection needle of the present invention, the flat plate portion is formed separately from the attaching portion to be attached to an injector or the like, and hence a shape of the flat plate portion can freely be designed irrespective of strength of the attaching portion. Consequently, the flat plate portion inclusive of the identifying shape portion can be formed into a shape by which a user easily identifies the blade face.

To attach and detach the injection needle to and from the injector, the needle base is often twisted to attach and detach the injection needle. In the injection needle of the present invention, the flat plate portion is disposed to the needle base, whereby force can easily be added to the needle base and the injection needle can easily be attached and detached to and from the injector or the like. Also in this case, the flat plate portion can be formed into a shape by which the user can easily add the force irrespective of the strength of the attaching portion.

Furthermore, in the injection needle of the first embodiment, when there is equipped a substantially cylindrical protector which is attached to the needle base so as to cover the needle tube and to receive the flat plate portion, it is preferable that the needle base be provided with a disc-shaped collar portion having substantially the same diameter as an outer diameter of the protector behind the flat plate portion. The identifying shape portion outward projects from the longitudinal side edge of the flat plate portion, and when the protector is equipped, it is also preferable that the identifying shape portion be interposed between the rear end portion of the protector and the collar portion to hold a space between both of them.

Usually, the injection needle often comprises the protector which covers the needle tube. The injection needle of the present invention has the disc-shaped collar portion, and the identifying shape portion becomes a projection interposed between the rear end portion of the protector and the collar portion. Accordingly, the projection makes a space between the protector and the collar portion, and therefore it becomes easy for a user to detach the protector from the injection needle. As described above, in the injection needle of the present invention, the identifying shape portion for the user to identify the direction of the blade face also performs a function to keep the space between the protector and the collar portion.

An injection needle of a second embodiment of the present invention comprises a needle tube having an oblique blade face formed at a tip thereof, and a needle base fixed to a base portion of the needle tube, wherein the needle base includes an attaching portion to be attached to an injector or the like and a flat plate portion of substantially flat plate shape disposed in front of the attaching portion, the flat plate portion is extended along a transverse direction orthogonal to upper and lower directions of the blade face, and on a surface in a direction of the blade face, there is disposed an identifying shape portion which enables a user to tactually identify a direction of the blade face.

Also in the injection needle of the second embodiment, the needle base is provided with the flat plate portion and the identifying shape portion in addition to the attaching portion. Accordingly, a user can easily identify the direction of the blade face by touching the identifying shape portion. Besides, since force can be easily added to the needle base, the user can easily attach and detach the injection needle to and from an injector and the like.

In the injection needle of the first and the second embodiment, it is preferable that the flat plate portion be equipped with an identifying indication by which the user can visually identify the direction of the blade face. The user always look at the injection needle before the injection, and if the identifying indication is disposed, the direction of the blade face of the needle tube can easily be visually identified. At the time of the injection, the user can further confirm tactually the direction of the blade face of the needle tube by the identifying shape portion.

An injection needle according to a third embodiment of the present invention comprises a needle tube having an oblique blade face formed at a tip thereof, and a needle base fixed to a base portion of the needle tube, wherein the needle base includes an attaching portion to be attached to an injector or the like and a grip portion disposed in front of the attaching portion, and the grip portion is formed so as to have a substantially isosceles triangle from a front angle, a vertex angle of the grip portion between two sides of equal length being an acute angle, the vertex angle being formed so as to conform with a direction of the blade face. As described above, the grip portion is formed into the substantially isosceles triangle, whereby a user can tactually identify the direction of the blade face when he grips the grip portion.

### Brief Description of the Drawings

Fig. 1 is an explanatory drawing illustrating the injection needle of the first embodiment;
Fig. 2 is an explanatory cross-sectional view that shows the protector accommodating the injection needle of the first embodiment;
Fig. 3 is a plan view that shows the protector being attached to the front side of the injection needle of the first embodiment;
Fig. 4 is a partially sectional view that shows the injection needle of the first embodiment being attached to an injector;
Fig. 5 is an explanatory drawing illustrating the injection needle of the second embodiment; and
Figs. 6a and 6b are explanatory drawings illustrating the injection needles of the third embodiment.

### Best Mode for Carrying Out the Invention

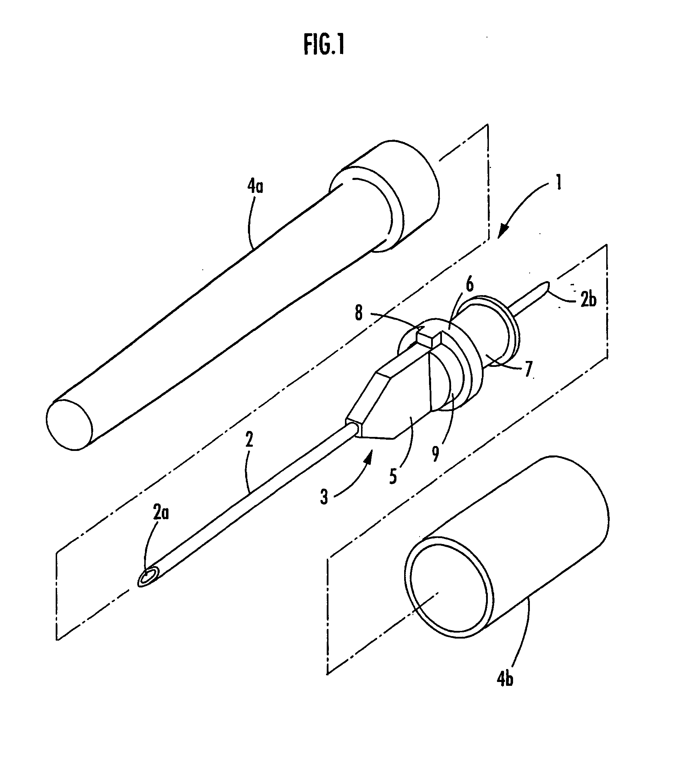

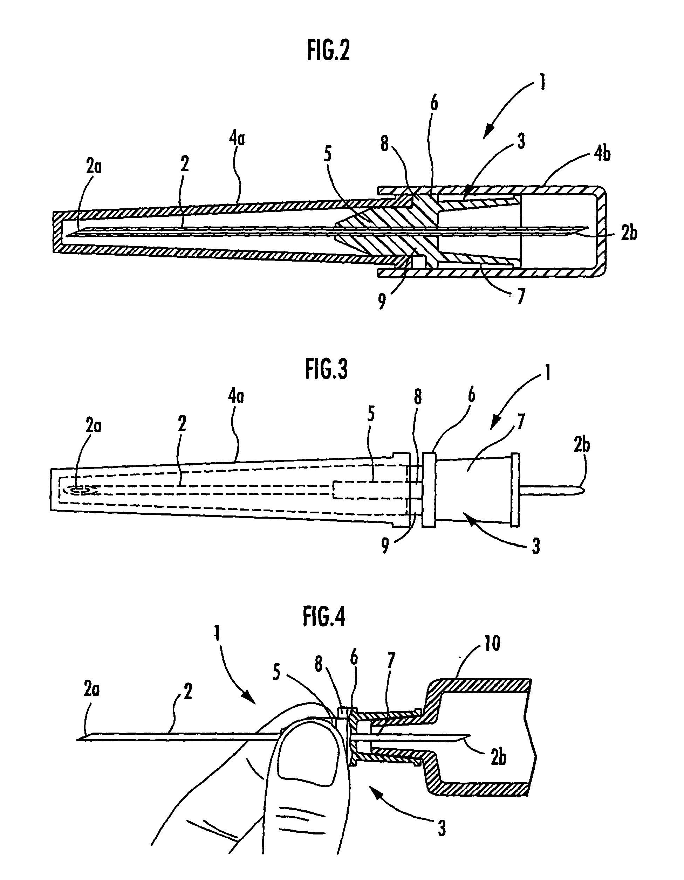

Next, examples of embodiments of an injection needle according to the present invention are described with reference to Fig. 1 to Fig. 6. First of all, an injection needle 1 of a first embodiment is explained with reference to Fig. 1 to Fig. 4. As shown in Fig. 1, the injection needle 1 of the first embodiment includes a needle tube 2 that sticks into an affected part, and a needle base 3 installed at a rear portion of the needle tube, the needle base 3 to be attached to an injector 10 (see Fig. 4). Protectors 4a and 4b are worn on the injection needle 1 in both front and rear sides respectively as shown in Fig. 2 such that the injection needle 1 is accommodated inside the protectors 4a and 4b. An injector 10 in the first embodiment is a so-called pre-filled syringe that is pre-filled with a drug solution.

The needle tube 2 is formed of stainless steel tube and has a blade face 2a formed by cutting along an oblique planar face at its tip. Furthermore, in the first embodiment, a rear end of the needle tube 2 sticks out rearward from the needle base 3, and a second blade face 2b is formed at the rear end.

The needle base 3 includes a flat plate portion 5 disposed in front of the needle base 3, a disc-shaped collar portion 6 attached to a rear end of the flat plate portion 5 and orthogonal to the axial direction of the needle tube 2, and an attaching portion 7 extending rearward from a rear end of the collar portion 6. The flat plate portion 5 further includes an after-mentioned identifying projection 8 (identifying shape portion). In front of the collar portion 6, there is formed a plug 9 which fits to the protector 4a and prevents foreign materials from getting mixed into the protector 4a. The above mentioned flat plate portion 5, collar portion 6, attaching portion 7, identifying projection 8, and plug 9 are integrally formed of synthetic resin.

The flat plate portion 5, formed into a thin plate as shown in Fig. 1, extends vertically along a direction to which the blade face 2a of the needle tube 2 opens. The identifying projection 8 sticks out of one side of a vertical side edge of the flat plate portion 5 up to the identical height with the collar portion 6. On the contrary, the identifying projection 8 is not prepared on the other side of the edge of the flat plate portion 5. A vertical height of the flat plate portion 5 is made substantially identical with an inside diameter of the protector 4a except at a location where the identifying projection 8 is formed. A diameter of the collar portion 6 is made substantially identical with that of the rear end portion of the protector 4a. A diameter of the attaching portion 7 increases rearward so as for the attaching portion 7 to fit a front end of the injector 10.

When the injection needle 1 with such a configuration is accommodated in the protectors 4a and 4b, a front side of the injection needle 1 is covered with the protector 4a and a rear side of the injection needle 1 is covered with the protector 4b as shown in Fig. 2. The vertical height of the flat plate portion 5 and an outer diameter of the plug 9 are substantially identical with the inside diameter of the protector 4a, and, when the protector 4a is put on, the flat plate portion 5 and the plug 9 fit to the inside circumferential surface of the protector 4a. Sticking outwardly from the flat plate portion 5, the identifying projection 8 is located in between the rear end portion of the protector 4a and the collar portion 6 to produce a predetermined space therebetween as shown in Fig. 2 and Fig. 3. Since an inside diameter of the protector 4b is made substantially identical with outer diameters of the rear end portion of the protector 4a and the collar portion 6, the protector 4b is worn covering the rear end portion of the protector 4a and the collar portion 6.

To detach the protectors 4a and 4b in such a condition, a user holds the protectors 4a and 4b with both hands, pulls them apart right and left, and removes the protector 4b. The user then holds the protector 4a and the collar portion 6 with both hands, and pulls them apart right and left. In the injection needle 1 of the first embodiment, as the identifying projection 8 existing in between the collar portion 6 and the rear end portion of the protector 4a produces the predetermined space, a user can easily pull both members apart.

Next, to attach the injection needle 1 to an injector 10, a user inserts the attaching portion 7 onto the front end of the injector 10 holding the flat plate portion 5. At this time, it is widely observed to twistingly insert the injection needle 1 into the injector 10 to surely attach the injection needle 1 to the injector 10. Since the flat plate portion 5 in the injection needle 1 of the first embodiment is formed to a thin plate, a user can easily add power to the injection needle 1.

When holding the needle base 3 to use the injector 10 with the injection needle 1 of the first embodiment, a user can easily identify the direction of the blade face 2a of the needle tube 2 by means of the identifying projection 8, and therefore, it is not necessary for the user to look at the blade face 2a to identify its direction. After use of the injector 10, the injection needle 1 should be detached from the injector 10. Since the injection needle 1 of the first embodiment has the flat plate portion 5 and therefore the injection needle 1 can easily be twisted, a user can easily remove the injection needle 1 from the injector 10.

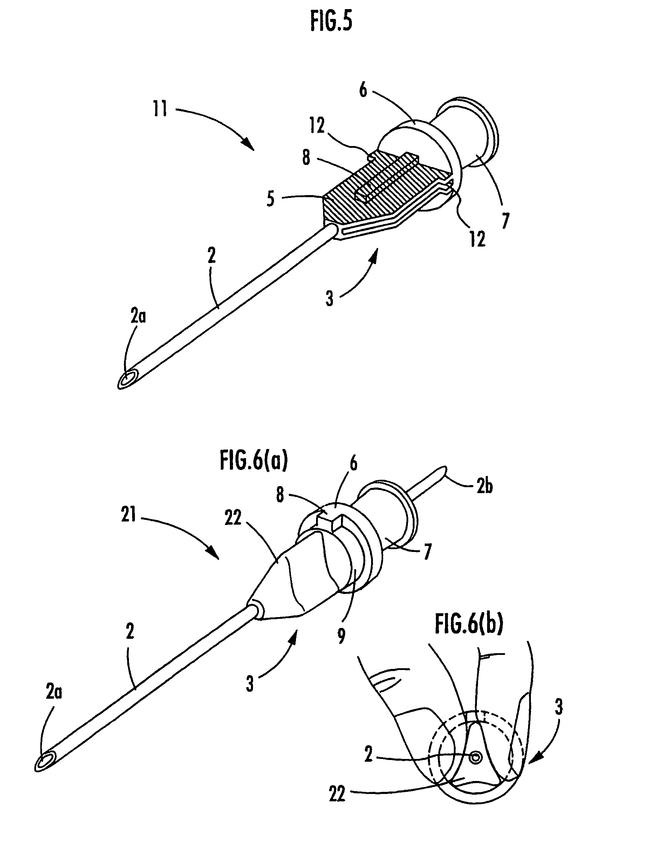

Next, an injection needle 11 of a second embodiment is described with reference to Fig. 5. The injection needle 11 of the second embodiment has a flat plate portion 5 which extends, as shown in Fig. 5, in a horizontal direction orthogonal to a direction of a blade face 2a. One surface of the flat plate portion 5 of the same side as the blade face 2a of a needle tube 2 is colored as an identifying indication (e.g. in red) and an identifying projection 8 is formed on the same surface of the flat plate portion 5 so that the identifying projection 8 sticks out along the direction of the blade face 2a. In this embodiment, the identifying projection 8 is formed lengthwise along an axial direction of the needle tube 2 on the surface of the flat plate portion 5. Because a needle base 3 of this embodiment is formed of synthetic resin, the needle base 3 can easily be colored.

In the second embodiment, projections 12 are formed, which abut a rear end of a protector 4a when the protector 4a is put onto both side edges of the flat plate portion 5. In the second embodiment, an attaching portion 7 is formed to be a cylindrical shape with a diameter increasing rearward so as to fit to a front end of a regular type injector, which is not filled with a drug solution (not shown, likewise hereafter). Other components are identical with those of the first embodiment and then identical reference numerals are given to each identical components in the drawing, omitting detailed descriptions on those components.

To attach the injection needle 11 of the second embodiment to an injector, a user inserts the attaching portion 7 onto the front end of the injector holding the flat plate portion 5. At this time, since the flat plate portion 5 is formed to a plate-like shape, the injection needle 11 can be surely inserted with twisting motion.

When the injector with the injection needle 11 of the second embodiment is used, a user can identify the direction of the blade face 2a of the needle tube 2 at a glance, because one surface of the flat plate portion 5 is colored. Furthermore, when an user holds the needle base 3 for injecting, the direction of the blade face 2a of the needle tube 2 can easily be identified with the identifying projection 8. In this embodiment, a user can easily identify the direction of the blade face 2a with the identifying projection 8, because the identifying projection 8 is formed lengthwise along the axial direction of the needle tube 2. As described above, using the injection needle 11 of the second embodiment, the direction of the blade face 2a can be identified easily and surely through visual and tactual discrimination. In addition, as the flat plate portion 5 is formed in the needle base 3, the injection needle can be easily attached and detached to and from the injector.

Next, an injection needle 21 of a third embodiment is described with reference to Fig. 6. A needle base 3 of the injection needle 21 of this embodiment has a grip portion 22 as shown in Figs. 6a and 6b, instead of a flat plate portion, the grip portion 22 being formed to have a substantially isosceles triangle cross-section from a front angle. In the grip portion 22, as shown in Fig. 6b, a vertex angle between two sides of equal length is made acute and a base is formed short. The grip portion 22 is formed as the acute vertex angle locates in the same direction as the blade face 2a as shown in Fig. 6a. Corners of the grip portion 22 are chamfered to be an arc shape such that a user may have a better touch when holding the grip portion 22. Other components are identical with those of the first embodiment and then identical reference numerals are given to identical components in Fig. 6, omitting detailed descriptions on those components.

To attach the injection needle 21 of the third embodiment to an injector, a user inserts an attaching portion 7 onto a front end of the injector holding the grip portion 22. At this time, as the grip portion 22 is formed to be substantially flat isosceles triangle shape, the injection needle 21 can be surely inserted into the injector with twisting motion.

When a user holds the needle base 3 to use the injector with the injection needle 21 of the third embodiment, the user can easily identify the direction of the blade face 2a of the needle tube 2 by the shape of the grip portion 22. As described above, using the injection needle 21 of the third embodiment, the direction of the blade face 2a can be tactually identified easily and surely. In addition, as the flat grip portion 22 is formed in the needle base 3, the injection needle can easily be attached and detached to and from the injector.

While the identifying projections 8 are adopted as identifying shape portion in the first and second embodiments described above, the identifying shape portion is not limited thereto, and a recess formed in a flat plate portion 5 may be used as an identifying shape portion. While a coloring is adopted as an identifying indication in the second embodiment described above, the identifying indication is not limited thereto, and patterns (including characters) clearly expressing the direction of the blade face may be drawn as an identifying indication.

### Industrial Applicability

The present invention can be utilized in an injection needle used for injection or for collection of blood or the like in a clinical site or another situation.

## Claims

1. An injection needle comprising a needle tube having an oblique blade face formed at a tip thereof, and a needle base fixed to a base portion of the needle tube, wherein
the needle base includes an attaching portion to be attached to an injector or the like, and a flat plate portion of substantially flat plate shape disposed in front of the attaching portion;
the flat plate portion is extended in accordance with upper and lower directions of the blade face;
an identifying shape portion which enables a user to tactually identify the direction of the blade face is formed on a side edge of the flat plate portion.

2. The injection needle according to claim 1, comprising a substantially cylindrical protector which is attached to the needle base so as to cover the needle tube and to receive the flat plate portion, the needle base being provided with a disc-shaped collar portion having substantially the same diameter as an outer diameter of the protector behind the flat plate portion, the identifying shape portion outward projecting from the longitudinal side edge of the flat plate portion, and when the protector is equipped, the identifying shape portion being interposed between the rear end portion of the protector and the collar portion to hold a space between both of them.

3. An injection needle comprising a needle tube having an oblique blade face formed at a tip thereof, and a needle base fixed to a base portion of the needle tube, wherein
the needle base includes an attaching portion to be attached to an injector or the like and a flat plate portion of substantially flat plate shape disposed in front of the attaching portion;
the flat plate portion is extended along a transverse direction orthogonal to upper and lower directions of the blade face; and on a surface in a direction of the blade face, there is disposed an identifying shape portion which enables a user to tactually identify a direction of the blade face.

4. The injection needle according to Claim 1, 2 or 3, wherein the flat plate portion is equipped with an identifying indication by which a user can visually identify the direction of the blade face.

5. An injection needle comprising a needle tube having an oblique blade face formed at a tip thereof, and a needle base fixed to a base portion of the needle tube, wherein
the needle base includes an attaching portion to be attached to an injector or the like and a grip portion disposed in front of the attaching portion, and the grip portion is formed so as to have a substantially isosceles triangle from a front angle; and
a vertex angle of the grip portion between two sides of equal length being an acute angle, the vertex angle being formed so as to conform with a direction of the blade face.
